Europäisches Patentamt

European Patent Office

Office européen des brevets

(11) Publication number: **0 538 518 A1**

# EUROPEAN PATENT APPLICATION

(21) Application number: **91309530.3**

(22) Date of filing: **16.10.91**

(51) Int. Cl.5: **C07C 2/66**, C07C 2/86, C07C 6/12, C07C 15/085

(43) Date of publication of application:
**28.04.93 Bulletin 93/17**

(84) Designated Contracting States:
**BE DE FR GB IT NL**

(71) Applicant: **Council of Scientific and Industrial Research**
**Rafi Marg**
**New Delhi 110 001(IN)**

(72) Inventor: **Rao, Bollapragad Seshagiri**
**National Chemical Laboratory**
**Pune-411008, Maharashtra(IN)**
Inventor: **Pradhan, Ajit Ramchandra**
**National Chemical Laboratory**
**Pune-411008, Maharashtra(IN)**
Inventor: **Ratnasamy, Paul**
**National Chemical Laboratory**
**Pune-411008, Maharashtra(IN)**

(74) Representative: **Collier, Jeremy Austin Grey et al**
**J.A.Kemp & Co., 14 South Square, Gray's Inn**
**London WC1R 5LX (GB)**

(54) An improved process for the production of cumene.

(57) An improved process is disclosed for the preparation of cumene. Cumene is prepared by reacting benzene with a propylating agent in the presence of a catalyst containing metal loaded Zeolite Beta in a reactor in the range of a temperature of 150 to 250°Cand a pressure of 1 to 35 atmospheres, the propyl and diisopropyl-benzene so formed are separated from the reactor effluent by conventional methods. The diisopropylbenzene is recycled back to the reactor. Simultaneous alkylation and transalkylation reactions occur in a single catalyst bed containing Zeolite Beta with a feed containing benzene, propylene and diisopropylbenzene. Cumenes are important chemical precursors in the production of detergents and polymers among others.

Rank Xerox (UK) Business Services
(3. 10 / 3.5x / 3.0. 1)

This invention relates to an improved process for the preparation of cumene by propylation of benzene with propylating agents in the presence of Zeolite Catalysts. More specifically, the present invention relates to a process for the propylation of benzene to isopropyl benzene in the presence of Zeolite Beta.

The alkylation of aromatics with alkylating agents in the presence of an alkylation catalyst is well known in the prior art to produce such monoalkylated products as ethyl benzene, isopropyl benzene or cumene and linear alkylated benzenes. Such monoalkyl aromatics are important chemical precursers in the production of detergents and polymers among others. Alkylation catalysts that are known to produce alkylaromatic compounds include the Fridel-Crafts catalyst, sulfuric acid, phosphoric acid, hydrofluoric acid, $AlCl_3$, clays, zeolites and amorphous silica-alumina.

The propylation of benzene to cumene using propylene as the alkylating agent with solid phophoric acid catalyst is in commercial practice for more than two decades. U.S. Patent 4,774,377 describes an alkylation-transalkylation process for the production of cumene using solid phophoric acid catalysts as the alkylation catalyst and Zeolite Mordenite as the transalkylation catalyst. U.S. Patent 4,395,372 describes an alkylation process using rare earth exchanged Faujasite type Zeolites as alkylation catalysts in the presence of sulphur dioxide. U.S. Patent 4,469,908 describes a process for alkylation of aromatic hydrocarbons using Zeolite ZSM-12. U.S. Patent 4,387,259 describes a process for the selective alkylation of aromatic hydrocarbons using ethylene/propylene as the alkylating agent over Zeolite ZSM-12.

Even though a variety of catalysts have been claimed to be useful in the propylation of benzene to cumene using propylene as the propylating agent, the only material to be used in the commercial practice are the solid phophoric acid catalysts. The use of isopropyl alcohol as the alkylating agent is not in commercial practice.

While catalysts based on solid phophoric acid are universally used in the process for cumene production, there are a number of limitations in their use in the above mentioned process:

(1) they are extremely sensitive to the moisture content in the propylene, the water content to be controlled precisely in the range of 200-500 ppm necessitating the installation of expensive process analysers and process control instruments. Too much water in the feed leads to the leaching of the phophoric acid catalyst, while too little water leads to the "frying" and deactivation of the catalyst. Morever isopropyl alcohol, an easily available alkylating agent cannot be used as a feedstock, since the water formed as a product will lead to the leaching and deactivation of the catalyst;

(2) a second limitation of the prior art phophoric acid catalyst is that the phophoric acid leached out during the process even while operating within the limits of the water content specified above leads to the corrosion of downstream equipment like distilation columns, used to separate cumene from the reactor effluent;

(3) a third limitation is that an acid sludge containing phophoric acid has to be periodically disposed off leading to environmental pollution;

(4) a fourth limitation of the solid phophoric acid catalyst is that it cannot be regenerated leading to a total catalyst life of about one year only;

(5) a fifth limitation of the solid phophoric acid catalyst is that it cannot transalkylate the diisopropyl benzenes (formed to an extent of 5-10% of the cumene in the process) to cumene.

While Zeolite catalysts based on Faujasite, Mordenite, ZSM-12 and ZSM-5 have been claimed to be useful in the propylation of benzene to cumene, no Zeolite based propylation process is in the commercial practice today. This is because of certain limitation of the above mention Zeolites in the process for the propylation of benzene to cumene. These limitations are (1)fast deactivation of the above mentioned Zeolites in the process tor propylation due to coke formation leading to low cycle lengths of less than six months, (2)low tolerence of Zeolites like Faujasites and Mordenites to even very low levels of water usually present in the propylene feedstock and (3)the high concentration of the diisopropyl benzenes in their product.

It is, hence, desirable to develop an improved process utilising catalysts which donot have the limitations of the above mentioned solid phophoric acid catalysts or Zeolites Faujasite, Mordenite, ZSM-12 and ZSM-5.

Accordingly, the present invention provides an improved process for the production of Cumene which comprises reacting benzene with propylating agent in the presence of a catalyst containing metal loaded Zeolite Beta in a reactor at a temperature in the range of 150 to 250ºC and a pressure of 1 to 35 atmospheres, separating the propyl and diisopropyl benzenes from the reactor effluent by conventional methods and recycling the diisopropylbenzene back to the said reactor.

Zeolite Beta is a crystalline aluminosilicate whose prerapation was first claimed in 1967 in U.S. Patent 3,308,069. Its framework structure has been elucideted only recently (Nature(London) volume 352, page 249, year 1988). Zeolite Beta is the only high silica Zeolite to have a fully 3-dimentional,12 ring pore system. Its framework structure is an intergrown hybrid of two distinct but closely related structures. The various factors that influence the preparation of Zeolite Beta have been reported in a recent article in the Journal ZEOLITES (page 46, volume 8, year 1988). Hence the preparation and structure of Zeolite Beta is well known in the prior art and donot form the subject of the present invention. The present invention describes the application and superiority of Zeolite Beta over prior art catalysts in the propylation of benzene to cumene in high yields.

In one embodiment of the present invention the metal loaded Zeolite Beta used as a catalyst in the propylation of benzene to cumene has a silica to alumina molar ratio in the framework above 20.

In another embodiment of the present invention the propylating agent is either propylene or propyl alcohol. It has been found that unlike prior art catalysts including other Zeolites like Faujasite, Mordenite, ZSM-5 and ZSM-12, Zeolite Beta retained its full activity and selectivity for the propylation reaction even in the presence of substantial quantities of water. This distinctive feature is a major advantage of the process of this invention over prior art processes. This ability of Zeolite Beta to function in the presence of water eliminates the corrosion and environmental problems associated with the use of solid phophoric acid catalyst in the prior art. In addition, precise control of the water content in the feedstock is also unnecessary in the process of the present invention.

In another embodiment of the present invention the diisopropyl benzenes in the reactor effluent are separated and recycled back to the alkylation reactor where they undergo transalkylation with benzene to yield cumene. This distinctive feature is another major advantage of the process of this invention. In the prior art processes using solid phophoric acid catalysts, transalkylation reactions cannot be done in the alkylation reactor. The diisopropyl benzene formed to an extent of 5-8% wt are usually diverted to the fuel pool leading to a lower utilisation of the raw materials, benzene and propylene in the production of cumene. The selectivity for the conversion of propylene to cumene in the process of present invention, for example, is around 98-99% wt compared to 93-95% in the prior art process using solid phosphoric acid catalysts.

The metal loaded Zeolite Beta used as a catalyst in the process of present invention is characterised by X-Ray diffraction pattern given in Table 1.

Table 1

| X-ray diffraction pattern of beta zeolite | |
|---|---|
| Interplannar spacing (A) | Relative intensity (I/Io) |
| 11.5 ± 0.4 | MS |
| 7.4 ± 0.2 | W |
| 6.6 ± 0.15 | W |
| 4.15 ± 0.1 | W |
| 3.97 ± 0.1 | VS |
| 3.54 ± 0.1 | W |
| 3.34 ± 0.1 | W |
| 3.03 ± 0.07 | W |
| 2.05 ± 0.05 | W |

Its adsorption potential for water and some hydrocarbon molecules is illustrated by the data in Table 2.

Table 2

Adsorption data for beta zeolite

Temp.    :  198°K

P/Po     :  0.5

| Solvent | Wt. % |
|---|---|
| H$_2$O | 21.3 |
| n-hexane | 19.0 |
| Cyclohexane | 10.5 |
| m-xylene | 25 |

These data were obtained in a conventional gravimetric adsorption apparatus at a relative partial pressure of the adsorbate of 0.5. It is desirable to incorporate the Zeolite Beta in another material more resistant to the temperature and other conditions employed in the process. Such matrix materials include synthetic or naturally ocuring substances as well as clay, silica, alumina or metal oxides. The relative proportion of Beta Zeolite and matrix binder material on anhydrous basis may vary widely with the Zeolite content ranging from between about 10 to 90% by weight.

The molar ratio of benzene to propylating agent employed may range between 20/1 to 1/1, more preferably 10/1 to 1/1. Reaction is suitably accomplished utilising a feed weight hourly space velocity (WHSV) of between 1 and 4. The later WHSV is based upon the total weight of active catalyst and binder thereof.

In one embodiment of the process of the present invention metals like sodium, potassium, calcium or barium are advantageously incorporated into Zeolite Beta to increase the selectivity to cumene. These metals may be incorporated into Zeolite Beta either during its synthesis or subsequently by ion-exchange after its incorporation with binder. It has also been found that a mixture of the above mentioned metals also has an advantageous effect in enhancing the selectivity of the catalyst. The total quantity of such added metals may vary between 0.1 to 5.0% wt.

The following examples will serve to illustrate the process of this invention without limiting the scope or utility thereof. The catalyst in all cases contained Zeolite Beta prepared as described in U.S. Patent no. 3,308,069

EXAMPLE 1

This example illustrates the preparation of the catalyst composite material used in the process of the present investigation. 70 gm of Zeolite Beta prepared as given in U.S. Patent 3,308,069 of 1967 and having silica to alumina mole ratio of 47 and Na content 0.1% wt was mixed with 30 gm of alumina hydrate and extruded into cylindrical pellets of 1/16" diameter. The extrudates were dried and calcined at 450°C in air for 10 hrs. The x-ray pattern and adsorption properties of Beta Zeolite are given in Tables 1 and 2 respectively shown above.

EXAMPLE 2

This example describes the process for the production of cumene using the catalyst prepared as described in example 1. 10 gm of the catalyst as prepared in example 1 is loaded in a fixed bed, down flow, high pressure, high temperature catalytic reactor. Benzene, propylene and propane were passed through the catalyst bed. The product analysis is repesented in Table 3 and compared with that of commercial solid phosphoric acid catalyst.

Table 3

Comparison of beta zeolite with conventional solid
phosphoric acid catalysts

|  | SPA | Zeolite |
|---|---|---|
| MR  B/PROPYLENE | 8 | 6.35 |
| WHSV | 1.25 | 3.5 |
| Temperature | 210 | 210 |
| Pressure | Atm. | Atm. |
| T.O.S  HR | 5 | 5 |
| Non-aromatics | 9.8 | 0.09 |
| Benzene | 77.00 | 78.42 |
| Toluene + $C_8$ A | 0.08 | 0.08 |
| Cumene | 11.80 | 19.40 |
| n-PB | 0.01 | 0.02 |
| $C_9$-$C_{11}$ A | - | 0.06 |
| $\Sigma$ DIPB | 1.00 | 1.83 |
| HA | 0.30 | 0.12 |
| % conversion $C_3$ | 80.40 | 9.93 |
| Sel. cumene % | 71.80 | 87.01 |
| $\dfrac{DIPB}{DIPB + IPB}$ X 100 | 7.81 | 8.62 |

EXAMPLE 3

This example (Table 4) indicates the effect of temperature on cumene production. The catalyst of example 1 is used. The catalyst contained 1.5% K, wt.

## Table 4

### Effect of temperature

B/P = 6.5, WHSV = 2.5, Press. = Atm.

| Temp. °C | 155 | 180 | 210 | 225 | 250 | 275 |
|---|---|---|---|---|---|---|
| Non-aromatics | 0.14 | 0.23 | 0.17 | 0.22 | 0.38 | 0.43 |
| Benzene | 79.50 | 77.70 | 78.40 | 78.60 | 79.0 | 79.20 |
| Tol. + $C_8$ A | 0.10 | 0.20 | 0.30 | 0.40 | 0.70 | 1.03 |
| Cumene | 16.93 | 20.27 | 19.86 | 19.10 | 17.89 | 17.29 |
| n-PB | 0.03 | 0.02 | 0.07 | 0.36 | 0.80 | 1.03 |
| $C_9$-$C_{11}$ A | 0.03 | 0.04 | 0.09 | 0.14 | 0.20 | 0.23 |
| $\Sigma$ DIPB | 2.87 | 1.27 | 1.01 | 0.97 | 0.84 | 0.79 |
| HA | 0.44 | 0.23 | 0.11 | 0.14 | 0.18 | 0.30 |
| % conversion $C_3$ | 99.88 | 99.78 | 99.84 | 99.79 | 99.63 | 99.61 |
| Sel. cumene % | 77.40 | 90.89 | 91.90 | 89.25 | 85.20 | 83.10 |
| $\dfrac{DIPB}{DIPB+IPB} \times 100$ | 14.45 | 5.90 | 4.84 | 4.83 | 4.48 | 4.37 |

EXAMPLE 4

The influence of benzene to propylene mole ratio on cumene formation is reported in the example (Table 5).

6

## Table 5

### Effect of mole ratio of benzene to propylene

210°C, WHSV = 2.5, Press. = Atm.

| Mole ratio | 4.05 | 6.23 | 7.67 | 10.77 | 13.20 |
|---|---|---|---|---|---|
| Non-aromatics | 0.08 | 0.06 | 0.06 | 0.04 | 0.05 |
| Benzene | 68.4 | 77.42 | 81.65 | 86.44 | 88.90 |
| Tol. $C_8$ A | 0.19 | 0.36 | 0.07 | 0.11 | 0.02 |
| Cumene | 26.50 | 20.55 | 16.95 | 12.85 | 10.62 |
| n-PB | 0.03 | 0.09 | 0.02 | 0.08 | 0.01 |
| $C_9$-$C_{11}$ A | 0.16 | 0.11 | 0.03 | 0.02 | - |
| $\leq$ DIPB | 4.18 | 1.19 | 1.14 | 0.40 | 0.37 |
| HA | 0.43 | 0.22 | 0.07 | 0.04 | 0.02 |
| % conversion $C_3$ | 99.94 | 99.96 | 99.96 | 99.98 | 99.98 |
| Sel. cumene % | 79.46 | 90.53 | 90.45 | 94.52 | 94.65 |
| $\frac{DIPB}{DIPB+IPB} \times 100$ | 13.62 | 5.47 | 6.30 | 3.02 | 3.37 |

### EXAMPLE 5

This example (Table 6) illustrates the selective cumene formation at different weight hourly space velocities.

Table 6

Effect of space velocity

Temp. = 215°C, B/P = 6.5, Press. = Atm.

| WHSV | 1 | 3 | 5 | 7 | 10 |
|---|---|---|---|---|---|
| Non-aromatics | 0.08 | 0.16 | 0.12 | 0.13 | 0.12 |
| Benzene | 78.08 | 76.62 | 78.09 | 77.04 | 77.83 |
| Tol. + $C_8$ | 0.29 | 0.39 | 0.22 | 0.12 | 0.09 |
| Cumene | 20.17 | 21.34 | 20.05 | 20.03 | 19.81 |
| n-PB | 0.07 | 0.11 | 0.03 | 0.03 | 0.02 |
| $C_9$-$C_{11}$ A | 0.08 | 0.12 | 0.09 | 0.14 | 0.10 |
| $\Sigma$ DIPB | 1.11 | 1.15 | 1.22 | 1.39 | 1.81 |
| $\Sigma$ HA | 0.10 | 0.11 | 0.15 | 0.20 | 0.12 |
| % conversion $C_3$ | 99.95 | 99.86 | 99.90 | 99.94 | 99.94 |
| Sel. cumene % | 92.02 | 91.27 | 91.51 | 87.24 | 89.35 |
| $\dfrac{DIPB}{DIPB+IPB} \times 100$ | 5.22 | 5.11 | 5.74 | 6.49 | 8.37 |

EXAMPLE 6

The variation of cumene content in the products obtained at different pressures is illustrated in this example (Table 7).

## Table 7

### Effect of pressure

Temp. = 215°C, WHSV = 5, B/P = 7

| | 1 | 10 | 18 | 25 |
|---|---|---|---|---|
| Pressure | | | | |
| Non-aromatics | 0.17 | 0.04 | 0.04 | 0.03 |
| Benzene | 78.40 | 78.77 | 79.04 | 79.56 |
| Tol. $C_8$ A | 0.30 | 0.12 | 0.03 | - |
| Cumene | 19.86 | 19.54 | 18.68 | 17.74 |
| n-PB | 0.07 | 0.04 | 0.02 | 0.01 |
| $C_9-C_{11}$ A | 0.09 | 0.08 | 0.03 | - |
| $\Sigma$ DIPB | 1.01 | 1.12 | 1.97 | 2.51 |
| HA | 0.11 | 0.25 | 0.17 | 0.15 |
| % conversion $C_3$ | 99.86 | 99.98 | 99.98 | 99.99 |
| Sel. cumene % | 91.94 | 92.04 | 89.12 | 86.79 |
| $\dfrac{DIPB}{DIPB+IPB} \times 100$ | 4.84 | 5.42 | 9.54 | 12.39 |

EXAMPLE 7

This example (Table 8) includes the results of the catalyst aging charecteristics with time on stream at atmospheric pressure. The catalyst contained 0.2% Na and 2.1% Ba, wt.

Table 8

Effect of time on stream

Temp. = 185°C, Press. = Atm., B/P = 6.5

| T.O.S (hrs) | 20 | 100 | 280 | 370 | 500 | 650 |
|---|---|---|---|---|---|---|
| Non-aromatics | 0.09 | 0.05 | 0.10 | 0.07 | 0.13 | 0.27 |
| Benzene | 77.32 | 77.07 | 77.72 | 79.35 | 80.06 | 81.12 |
| Tol. + $C_8$ A | 0.21 | 0.06 | 0.05 | 0.10 | 0.03 | 0.05 |
| Cumene | 20.69 | 20.67 | 19.41 | 18.28 | 17.82 | 16.70 |
| n-PB | 0.04 | 0.02 | 0.01 | 0.11 | 0.02 | 0.15 |
| $C_9$-$C_{11}$ A | 0.09 | 0.05 | 0.02 | 0.04 | 0.03 | 0.05 |
| $\Sigma$ DIPB | 1.36 | 1.93 | 2.57 | 1.94 | 1.91 | 1.60 |
| HA | 0.20 | 0.11 | 0.11 | 0.06 | 0.05 | 0.03 |
| % conversion $C_3$ | 99.93 | 99.96 | 99.92 | 99.93 | 99.89 | 99.75 |
| Sel. cumene % | 91.23 | 90.14 | 90.19 | 88.52 | 89.10 | 88.92 |
| $\dfrac{\text{DIPB}}{\text{DIPB+IPB}}$ X 100 | 6.17 | 8.54 | 11.69 | 9.59 | 9.68 | 8.74 |

EXAMPLE 8

The variation of benzene to propylene mole ratio at 25 kg/cm$^2$ pressure is included in this example (Table 9).

Table 9

Effect of mole ratio

Temp. $250^{\circ}$C, 25 Atm., WHSV = 5

| Mole ratio | 3.1 | 6.0 | 7.5 | 14.1 |
|---|---|---|---|---|
| Non-aromatics | 0.13 | 0.03 | 0.01 | 0.01 |
| Benzene | 60.27 | 76.52 | 80.55 | 87.86 |
| Tol. + $C_8$ A | 0.03 | 0.06 | 0.05 | 0.03 |
| Cumene | 30.26 | 22.02 | 18.34 | 11.62 |
| n-PB | 0.05 | 0.12 | 0.12 | 0.14 |
| $C_9$-$C_{11}$ A | 0.11 | 0.04 | 0.03 | 0.01 |
| $\xi$ DIPB | 8.45 | 1.14 | 0.84 | 0.32 |
| HA | 0.57 | 0.05 | 0.09 | 0.01 |
| % conversion $C_3$ | 99.90 | 99.99 | 100 | 100 |
| Sel. cumene % | 76.19 | 93.78 | 74.29 | 95.72 |
| $\dfrac{\text{DIPB}}{\text{DIPB+IPB}}$ X 100 | 21.83 | 4.92 | 4.38 | 2.68 |

EXAMPLE 9

The changes in the product distribution due to changes in the weight hourly space velocity is illustrated in this example (Table 10).

Table 10

Effect of space velocity

210°C, 25 Atm., B/P = 6.

| WHSV | 3.5 | 5 | 7.5 | 10 | 5 |
|---|---|---|---|---|---|
| Non-aromatics | 0.01 | 0.01 | 0.03 | 0.02 | 0.04 |
| Benzene | 75.88 | 76.42 | 76.62 | 77.26 | 77.41 |
| Tol. + $C_8$ | 0.07 | 0.02 | 0.02 | 0.01 | 0.01 |
| Cumene | 22.61 | 21.47 | 21.23 | 20.45 | 20.12 |
| n-PB | 0.07 | 0.02 | 0.02 | 0.01 | 0.01 |
| $C_9$-$C_{11}$ | 0.04 | 0.01 | 0.01 | 0.01 | - |
| $\Sigma$ DIPB | 1.24 | 1.31 | 2.02 | 2.21 | 2.40 |
| HA | 0.07 | 0.11 | 0.05 | 0.02 | 0.02 |
| % conversion $C_3$ | 100 | 100 | 99.99 | 100 | 99.98 |
| $\dfrac{DIPB}{DIPB+IPB} \times 100$ | 5.20 | 5.75 | 8.69 | 9.75 | 10.66 |

EXAMPLE 10

This example (Table 11) illustrates the influence of temperature when using a catalyst containing Beta Zeolite of silica to alumina ratio 85. The catalyst contained 1.2% Ca and 0.5% Ba, wt.

Table 11

Effect of temperature

B/P = 6, WHSV = 5, Press. = 25 Atm.

| Temp. $^{\circ}$C | 195 | 210 | 230 | 250 | 280 |
|---|---|---|---|---|---|
| Non-aromatics | 0.04 | 0.01 | 0.03 | 0.04 | 0.10 |
| Benzene | 77.90 | 76.42 | 76.52 | 76.46 | 76.24 |
| Tol. + $C_8$ | 0.01 | 0.02 | 0.06 | 0.15 | 0.34 |
| Cumene | 18.92 | 22.10 | 22.02 | 21.35 | 20.48 |
| n-PB | 0.02 | 0.01 | 0.12 | 0.62 | 1.28 |
| $C_9$-$C_{11}$ A | - | 0.02 | 0.04 | 0.09 | 0.18 |
| $\leq$ DIPB | 3.03 | 1.31 | 1.17 | 1.15 | 1.08 |
| HA | 0.01 | 0.11 | 0.05 | 0.10 | 0.25 |
| % conversion $C_3$ | 99.98 | 99.99 | 99.99 | 99.98 | 99.93 |
| Sel. cumene % | 85.61 | 93.65 | 93.70 | 90.70 | 85.60 |
| $\dfrac{\text{DIPB}}{\text{DIPB+IPB}} \times 100$ | 13.80 | 5.60 | 9.50 | 5.11 | 5.01 |

EXAMPLE 11

This example (Table 12) illustrates the regenerability of the catalyst. The catalyst after deactivation was regenerated and tested for its activity.

## Table 12

### Catalyst regenerability

B/P = 7.5; $210^{\circ}C$, WHSV = 3.0, 25 Atm.

| Products, % wt. | Cycle 1 | After regen; |
|---|---|---|
| Aliphatics | 0.04 | 0.02 |
| Benzene | 79.6 | 79.5 |
| Tol. + EB | 0.07 | 0.04 |
| Cumene | 19.1 | 19.3 |
| n-PB | 0.09 | 0.08 |
| $C_9-C_{11}$ | 0.05 | 0.04 |
| $\Sigma$ DIPB | 0.98 | 1.05 |
| HA | 0.12 | 0.15 |

## EXAMPLE 12

This example (Table 13) illustrates the simulteneous occurence of both alkylation and transalkylation reactions in a single catalyst bed containing Zeolite Beta with a feed containing benzene, propylene and diisopropyl benzenes. The silica to alumina ratio in the Zeolite was 34. The catalyst contained 0.5% Na, 0.8% Ca and 0.1% K, wt.

## Table 13

### Simultaneous alkylation and transalkylation

B/P = 7.0, 240°C, 25 Atm.

The feed contained 2 % wt. of diisopropyl benzene.

### Products, % wt.

| | |
|---|---|
| Aliphatics | 0.1 |
| Benzene | 80.05 |
| Tol. + EB | 0.07 |
| Cumene | 18.55 |
| n-PB | 0.2 |
| $C_9-C_{11}$ | 0.02 |
| % DIPB | 1.02 |
| HA | 0.04 |

This example (Table 14) shows the use of isopropanol as alkylating agent in place of propylene.

Table 14

Comparison of propylene with isopropanol as alkylating agents

B/P = 7.5, WHSV = 2.5, Atm. press.

| Alkylating agent | Propylene | Propylene | Isopropanol |
|---|---|---|---|
| Temp. °C | 180 | 205 | 205 |
| Non-aromatics | 0.23 | 0.17 | 0.12 |
| Benzene | 78.17 | 78.37 | 78.46 |
| Tol. + $C_8$ | 0.24 | 0.30 | 0.26 |
| Cumene | 19.89 | 19.86 | 19.58 |
| n-PB | 0.02 | 0.07 | 0.03 |
| $C_9$-$C_{11}$ A | 0.06 | 0.09 | 0.11 |
| $\sum$ DIPB | 1.22 | 1.01 | 1.27 |
| HA | 0.17 | 0.11 | 0.15 |

Claims

1.  An improved process for the preparation of cumene which comprises reacting benzene with a propylating agent in the presence of a catalyst containing metal loaded Zeolite Beta in a reactor at a temperature in the range of 150 to 250°C and a pressure in the range of 1 to 35 atmospheres, separating propyl and diisopropylbenzene from the reactor by conventional methods and recycling the diisopropylbenzene back to the said reactor.

2.  The process as claimed in claim 1 wherein the propylating agent is selected from propylene and propyl alcohol.

3.  The process as claimed in claim 1 wherein the molar ratio benzene and propylating agent in the mixture is between 1 and 20, preferably between 1 and 10.

4.  The process as claimed in claim 1 wherein the Zeolite Beta used has a silica to alumina mole ratio above 20.

5.  The process as claimed in claim 1 wherein the metal loaded Zeolite Beta is from one or more of the metal selected from the group sodium, calcium, potassium and barium.

6.  The process as claimed in claim 1 wherein the amount of metal loaded into the Zeolite Beta ranges from 0.1 to 0.5% by weight.

7.  The process as claimed in claim 1 wherein the metal loaded Zeolite Beta contains as binder clay, silica, alumina, metal oxides and the like.

8.  The process as claimed in claim 7 wherein the amount of the binder ranges from 10-90% by weight.

9. The process as claimed in claim 1 wherein the reaction is effected at a weight hourly space velocity (WHSV) of 1 to 4.

## EUROPEAN SEARCH REPORT

European Patent Office

Application Number

EP 91 30 9530

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 891 458 (ROBERT A. INNES ET AL.)<br>* claims 1-15,19-45; examples 4-5,7-8 *<br>--- | 1-3,5-9 | C07C2/66<br>C07C2/86<br>C07C6/12<br>C07C15/085 |
| X | EP-A-0 432 814 (ENIRICERCHE S.P.A.)<br>* claims; examples 1,3-7 *<br>--- | 1-9 | |
| A | EP-A-0 012 504 (MOBIL OIL)<br>* claims; examples *<br>----- | 1-9 | |
| | | | TECHNICAL FIELDS SEARCHED (Int. Cl.5) |
| | | | C07C |

The present search report has been drawn up for all claims

| Place of search | Date of completion of the search | Examiner |
|---|---|---|
| THE HAGUE | 23 JUNE 1992 | ZERVAS B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)